# EUROPÄISCHE PATENTSCHRIFT

(11) **EP 1 367 965 B1**
(45) Veröffentlichungstag und Bekanntmachung des Hinweises auf die Patenterteilung: **18.07.2007**
(21) Anmeldenummer: 02712925.3
(22) Anmeldetag: 26.02.2002
(51) Int. Cl.: A61F 2/34, A61F 2/46

(54) **SETZINSTRUMENT ZUM EINBRINGEN EINER SPREIZSCHALE**
POSITIONING INSTRUMENT FOR INSERTING AN EXTENSION SHELL
INSTRUMENT DE POSITIONNEMENT DESTINE A L'INSERTION D'UNE COQUILLE D'ECARTEMENT

(30) Priorität: 15.03.2001 DE 10112527
(43) Veröffentlichungstag der Anmeldung: 10.12.2003
(73) Patentinhaber: Mathys Medizinaltechnik AG, 2544 Bettlach (CH)
(72) Erfinder: COTTING, Anton, CH-2540 Grenchen (CH); CHRISTEN, Peter, CH-25245 Selzach (CH); DELFOSSE, Daniel, CH-3018 Bern (CH)
(74) Vertreter: Körfer, Thomas
(86) Internationale Anmeldenummer: PCT/EP2002/002039
(87) Internationale Veröffentlichungsnummer: WO 2002/074203

(56) Entgegenhaltungen:
- EP-A- 0 169 978
- FR-A- 2 710 522
- GB-A- 2 299 758
- US-A- 5 976 148

## Beschreibung

Die Erfindung betrifft ein Setzinstrument zum Einbringen einer Spreizschale in einer menschlichen oder tierischen Knochen. Die Erfindung betrifft insbesondere ein Setzinstrument zum Einbringen einer Spreizschale, die Teil einer Hüftgelenksendoprothese ist, in den Beckenknochen.

Aus der EP 0 242 633 B1 ist eine Spreizpfanne als Endoprothese für eine Hüftgelenkspfanne zur zementfreien Verankerung in einem Beckenknochen bekannt. Für diese bekannte Spreizpfanne wird die Spreizschale mit Hilfe eines Werkzeuges zunächst elastisch zusammengepreßt und dann in axialer Richtung in eine operativ geschaffene Ausnehmung im Beckenknochen eingesetzt. Als bisher übliches Werkzeug dient eine geeignete Zange oder dergleichen.

Die bekannte Methode zum Einbringen einer Spreizschale mittels einer Zange oder dergleichen hat jedoch mehrere Nachteile. Da das Einbringwerkzeug die Spreizschale seitlich umfaßt, besteht beim Einbringen der Spreizschale die Gefahr, daß das umliegende Knochengewebe beschädigt wird. Ferner gestaltet sich das Entfernen des Einbringwerkzeuges schwierig, da aufgrund des angrenzenden Knochenmaterials das Einbringwerkzeug nur teilweise geöffnet werden kann, so daß beim Herausziehen die Spreizschale leicht verrutscht wird und/oder das angrenzende Knochenmaterial beschädigt wird. Außerdem muß der operierende Arzt mit dem Einbringwerkzeug in unmittelbarer Nähe des Beckenknochens hantieren, wodurch die erforderliche Einbringkraft schwer aufzubringen ist und ein ergonomisches Arbeiten nicht möglich ist.

In der Praxis ist das Einbringen einer Spreizschale daher aufwendig und mit erheblichen Risiken belastet. Außerdem konnten die zum vollständigen Einbringen der Spreizschale in den Knochen in axialer Richtung erforderlichen Druckkräfte, insbesondere während des Entspannens der Spreizschale, bisher nur ungenügend aufgebracht werden.

Aus der US-A-5,976,148 ist eine Vorrichtung bekannt, die eine Befestigungseinrichtung zum Befestigen der Spreizschale und eine Verstelleinrichtung zum axialen Verstellen des Tellerelements gemäß Oberbegriffs des Anspruchs 1 aufweist.

Aufgabe der Erfindung ist es daher, ein Setzinstrument zu schaffen, das das Einbringen in den Knochen erleichtert und mit dem ein zuverlässiger Sitz der implatierten Spreizschale erreicht werden kann, wobei beim Entfernen des Setzinstruments eine nachträgliche Dejustierung der Spreizschale bzw. eine Beschädigung des umgebenden Knochenmaterials verhindert ist.

Die Aufgabe wird durch ein Setzinstrument mit den Merkmalen des Anspruchs 1 gelöst. Vorteilhafte Weiterbildungen der Erfindung sind durch die in den Unteransprüchen angegebenen Maßnahmen möglich.

Das erfindungsgemäße Setzinstrument hat den Vorteil, daß mit dem Tellerelement des Setzinstrumentes die Spreizschale zusammengedrückt, d. h. gespannt werden kann, ohne daß das Setzinstrument die Spreizschale umfänglich umfaßt. Dadurch wird verhindert, daß das Setzinstrument beim Einbringen der Spreizschale in den Knochen das umgebende Knochenmaterial beschädigt. Da das Setzinstrument dadurch auch die Spreizschale im entspannten, d. h. gespreizten, Zustand nicht umfaßt, kann das Setzinstrument besonders einfach entfernt werden. Außerdem ragt das Setzinstrument radial nicht über die radial am weitesten vorstehenden Elemente der gespannten Spreizschale hinaus, so daß das Einbringen des Setzinstruments zusammen mit der Spreizschale in jedem Fall möglich ist.

Erfindungsgemäß umfaßt das Tellerelement drehbar gelagerte Walzen, die beim Spannen der Spreizschale an den abgeschrägten Flächen der Spreizschale abrollen. Dadurch wird das Setzinstrument geschont, da ein Abrieb im Bereich des auf die Spreizschale einwirkenden Tellerelements verhindert wird. Außerdem werden die Reibungskräfte beim Spannen und Entspannen der Spreizschale vermindert, wodurch geringere Spann- und Entspannkräfte erforderlich sind, so daß zum Verstellen des Tellerelementes des Setzinstrumentes geringere Verstellkräfte erforderlich sind. Anstelle der Walzen können auch Rollen, Rollkörper, Wälzkörper oder dergleichen zum Einsatz kommen, wobei die Walzen bzw. die Wälzkörper den Vorteil haben, daß die Kraftübertragung zum Spannen der Spreizschale über eine größere Fläche erfolgt.

In vorteilhafter Weise umfaßt die Verstelleinrichtung ein Gewinderad, das mittels einer Distanzhülse auf das Tellerelement einwirkt. Durch das Gewinderad läßt sich die Distanzhülse in einfacher Weise vorspannen und nach dem Einbringen wieder entspannen, wobei durch die Distanzhülse ein einfacheres Arbeiten, insbesondere in Entfernung zum Patienten, ermöglicht wird. Die Einwirkung des Tellerelementes auf die Distanzhülse kann dabei durch eine Hubübersetzungseinrichtung vermittelt werden, die das Spannen und Entspannen der Spreizschale mit geringerer Kraft ermöglicht.

Die Erfindung wird nachfolgend anhand eines Ausführungsbeispiels unter Bezugnahme auf die Zeichnung näher erläutert. In der Zeichnung zeigen:
- Fig. 1: ein Ausführungsbeispiel eines Setzinstrumentes zusammen mit einer Spreizschale;
- Fig. 2: einen Ausschnitt des in Fig. 1 gezeigten Grundkörpers des Setzinstruments;
- Fig. 3: den in Fig. 1 mit III bezeichneten Ausschnitt;
- Fig. 4A: die Übertragungshülse des in Fig. 1 dargestellten Setzinstruments im Detail;
- Fig. 4B: die in Fig. 4A dargestellte Übertragungshülse in einer Vorderansicht aus der in Fig. 4A mit IV B bezeichneten Richtung;
- Fig. 5A: das Gewinderad des in Fig. 1 dargestellten Setzinstruments im Detail; und
- Fig. 5B: das in Fig. 5A dargestellte Gewinderad in einer Vorderansicht aus der in Fig. 5A mit V B bezeichneten Richtung.

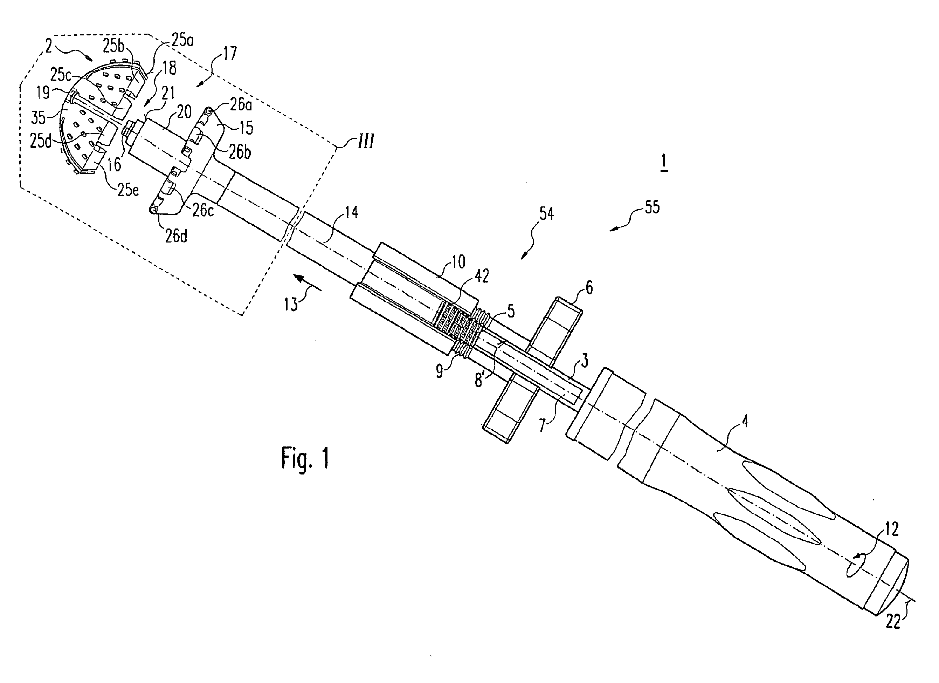

Fig. 1 zeigt ein Ausführungsbeispiel des erfindungsgemäßen Setzinstruments 1 zusammen mit einer Spreizschale 2. Das Setzinstrument 1 dient zum Einbringen der Spreizschale 2 in einen menschlichen oder tierischen Knochen. Insbesondere dient das Setzinstrument 1 zum Einbringen einer Spreizschale 2, die Teil einer Hüftgelenkendoprothese ist, in den Beckenknochen.

Das Setzinstrument 1 umfaßt einen Grundkörper 3, an dem ein Griff 4 befestigt ist. Der Grundkörper 3 weist ein als Trapezgewinde ausgebildetes Gewinde 5 auf, auf das ein Gewinderad 6 aufgebracht ist. Um das Aufbringen des Gewinderads 6 auf den Grundkörper 3 zu ermöglichen, sind an dem Grundkörper 3 zwei gegenüberliegende Flächen 7, 8 ausgebildet, wobei in der Fig. 1 nur die Fläche 7 dargestellt ist, entlang der das Gewinderad 6 über die an dem Gewinderad 6 ausgebildete nutförmige Aussparung aufschiebbar ist. An dem Gewinderad 6 ist ein Außengewinde 9 ausgebildet, das in ein an einer Übertragungshülse 10 ausgebildetes Innengewinde 42 eingreift. Die Übertragungshülse 10 ist axial geführt, so daß eine Drehung der Übertragungshülse 10 um die Längsachse 12 des Setzinstruments 1 blockiert ist.

Bei einer Drehung des Gewinderads 6 im Uhrzeigersinn 12 schraubt sich das Gewinderad 6 in die Übertragungshülse 10, so daß die Übertragungshülse 10 relativ zu dem Gewinderad 6 auf das Gewinderad 6 zu gezogen wird, wodurch ein von dem Gewinde 9 vermittelter Hub der Übertragungshülse 10 gegeben ist. Außerdem schraubt sich das Gewinderad 6 zunehmend auf das Gewinde 5, so daß ein relativ zu dem Griff 4 weggerichteter Hub des Gewinderades 6 gegeben ist. Der Hub der Übertragungshülse 10 ist daher entgegengesetzt zu dem Hub, der das Gewinderad 6 betrifft. Da die Steigung des Gewindes 5 des Grundkörpers 3 größer ist als die Steigung des Außengewindes 9 des Gewinderades 6, ergibt sich ein resultierender Hub in Einbringrichtung 13 der Übertragungshülse 10 relativ zu dem Grundkörper 3. Diesen resultierenden Hub überträgt die Übertragungshülse 10 auf eine Distanzhülse 14, wodurch ein Tellerelement 15 des Setzinstruments 1 in der Verstell- bzw. Einbringrichtung 13 axial verstellt wird.

Umgekehrt erfolgt durch eine Drehung des Gewinderades 6 entgegen dem Uhrzeigersinn 12 eine Verstellung des Tellerelements 15 entgegen der Einbringrichtung 13.

Der Grundkörper 3 weist außerdem einen Gewindestift 16 auf, der am oberen Ende 17 des Setzinstruments 1 vorgesehen ist. Der Gewindestift 16 ist Teil einer Befestigungseinrichtung 18, die zum Befestigen der Spreizschale 2 an dem Setzinstrument 1 dient. Dabei weist die Spreizschale 2 ein Gewinde 19 auf, mit dem sich diese auf den Gewindestift 16 aufschrauben läßt. Durch Verstellen des Tellerelements 15 in Einbringrichtung 13 wird die aufgeschraubte Spreizschale 2 gespannt. Das Tellerelement 15 weist eine Hülse 20 auf, wobei durch Anschlagen der Stirnfläche 21 der Hülse 20 an der Innenfläche der Spreizschale 2 im Bereich des Gewindes 19 die maximale Verstellung des Tellerelements 15 und damit die maximal auf die Spreizschale 2 einwirkende Spannkraft begrenzt ist.

Zum Spannen der Spreizschale 2 wirkt das Tellerelement 15 auf abgeschrägte Flächen der Spreizschale 2 ein, wobei in der Fig. 1 die abgeschrägten Flächen 25a bis 25e dargestellt sind. Um die Abnützung des Tellerelements 15 des Setzinstruments 1 zu verringern, weist das Tellerelement 15 mehrere umfänglich verteilte Walzen auf, von denen in der Fig. 1 die Walzen 26a bis 26d dargestellt sind. Beim Spannen der Spreizschale 2 rollen die Walzen 26a bis 26d an den entsprechenden abgeschrägten Flächen 25a bis 25e ab.

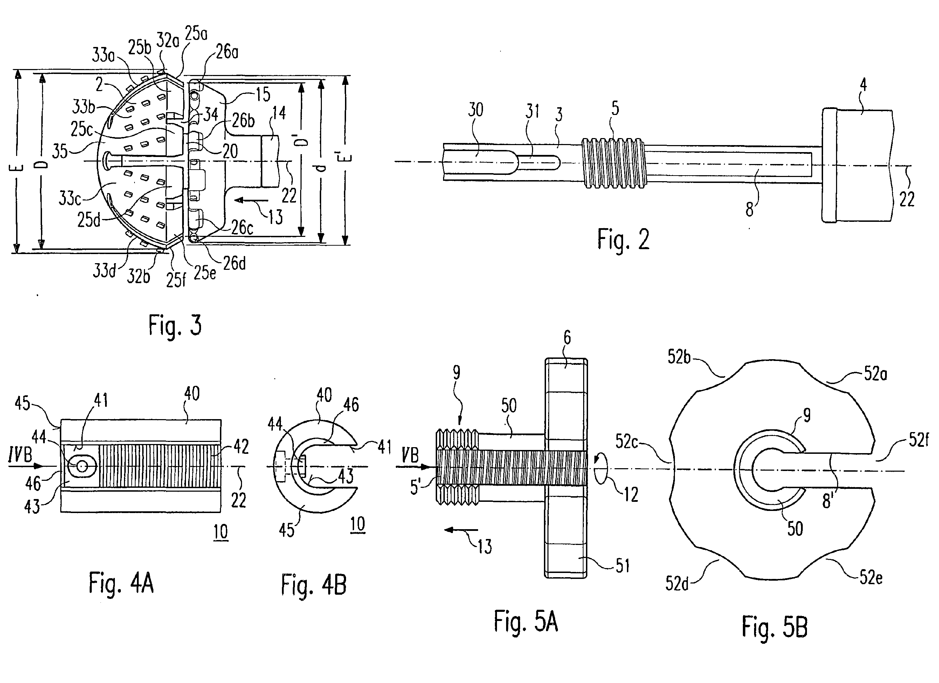

Fig. 2 zeigt einen Ausschnitt des in Fig. 1 dargestellten Grundkörpers 3 des Setzinstruments 1. Bereits beschriebene Elemente sind in dieser und in allen anderen Figuren mit übereinstimmenden Bezugszeichen versehen, wodurch sich eine wiederholende Beschreibung erübrigt.

Fig. 2 zeigt eine Ansicht auf den Grundkörper des Setzinstrument 1, die gegenüber der in Fig. 1 dargestellten Ansicht um 180° bezüglich der Längsachse 22 gedreht ist. Daher ist von den beiden Flächen 7, 8, die an dem Grundkörper 3 ausgebildet sind, in der Fig. 2 die der Fläche 7 gegenüberliegende Fläche 8 dargestellt. Der Grundkörper 3 weist außerdem eine Aussparung 30, in die die Übertragungshülse 10 eingreift, und eine daran anschließende Aussparung 31 auf. Durch das Eingreifen der Übertragungshülse 10 in die Aussparung 30 wird eine Drehung der Übertragungshülse 10 um die Längsachse 22 des Grundkörpers 3 zumindest bis auf ein gewisses Spiel blockiert, so daß eine axiale Führung der Übertragungshülse 10 gegeben ist, da die Aussparung 30 als Längsnut ausgebildet ist.

Die Fig. 3 zeigt den in Fig. 1 mit III bezeichneten Ausschnitt des Setzinstrument 1 und die Spreizschale 2 im aufgeschraubten aber ungespannten Zustand.

Zum Spannen der Spreizschale 2 wird das Tellerelement 15 in der Einbringrichtung 13 relativ zu der an der Befestigungseinrichtung 18 befestigten Spreizschale 2 verstellt. Dabei wirkt das Tellerelement 15 über die Walzen 26a bis 26d auf die abgeschrägten Flächen 25a bis 25f ein. Um die Abnützung des Tellerelements 15 zu verringern und um das Spannen der Spreizschale 2 zu erleichtern, rollen die Walzen 26a bis 26d an den ihnen gegenüberliegenden Flächen 25a bis 25f ab. Hierzu läßt sich das Tellerelement 15 um die Längsachse 22 drehen, um die Walzen 26a bis 26d in geeignete Positionen zu bringen. In dem dargestellten Ausführungsbeispiel wird die Walze 26a gegenüberliegend zu der abgeschrägten Fläche 25a angeordnet, die Walze 26b wird gegenüberliegend zu der abgeschrägten Fläche 25c angeordnet, die Walze 26c wird gegenüberliegend zu der abgeschrägten Fläche 25e angeordnet und die Walze 26d wird gegenüberliegend zu der abgeschrägten Fläche 25f angeordnet. Dadurch liegen die Walzen 26a bis 26d über ihre gesamte Längsrichtung an den abgeschrägten Flächen 25a bis 25f an, so daß eine gleichmäßige Kraftübertragung gewährleistet ist.

Alternativ können die Walzen 26a bis 26d auch jeweils zwischen den beiden abgeschrägten Flächen 25a - 25f eines Segmentes 33a - 33d zum Spannen der Spreizschale 2 angeordnet werden. In Bezug auf das Segment 33b ist dann die Walze 26b gegenüberliegend zu dem Zwischenraum 34 zwischen den beiden Flächen 25b, 25c angeordnet, so daß die Walze 26b jeweils teilweise an der Fläche 25b und teilweise an der Fläche 25c anliegt. Die Walze 26b liegt dadurch im wesentlichen parallel zu der Drehachse, um die das Segment 33b beim Spannen und Entspannen in geringem Umfang verdreht wird. Da die Walze 26b dabei mittig am Ende des Segments 33b angreift, ist eine vorteilhafte Kraftübertragung gegeben, die ein radialsymmetrisches Spannen des Segmentes 33b ermöglicht.

Im entspannten Zustand hat die Spreizschale 2, abgesehen von einzelnen Vorsprüngen, wie z. B. den Vorsprüngen 32a und 32b, den Durchmesser D. Das heißt die Projektion des Grundkörpers 35 der Spreizschale 2 entlang der Längsachse 22 in Einbringrichtung 13 entspricht im Wesentlichen einem Kreis mit Durchmesser D. Das Tellerelement 15 weist im Wesentlichen den Durchmesser d auf, so daß die Projektion des Tellerelements 15 in Einbringrichtung 13 im Wesentlichen einem Kreis mit Durchmesser d entspricht. Da der Durchmesser d des Tellerelements 15 kleiner ist als der Durchmesser D des Grundkörpers 35 der Spreizschale 2, ragt die in Einbringrichtung 13 betrachtete Projektion des Tellerelements 15 nicht über die in Einbringrichtung 13 betrachtete Projektion der Spreizschale 2 im ungespannten Zustand hinaus.

Der Durchmesser d des Tellerelements 15 ist allerdings sogar um soviel kleiner als der Durchmesser D der Spreizschale 2 im ungespannten Zustand gewählt, daß die Projektion des Tellerelementes 15 auch nicht über die in Einbringrichtung 13 betrachtete Projektion der radial vorstehenden Vorsprünge 32a, 32b der Spreizschale 2 im gespannten Zustand hinausragt. Der Durchmesser d des Tellerelementes 15 ist also kleiner als der Durchmesser E' der gespannten Spreizschale 2 bezogen auf die radial am weitesten vorstehenden Vorsprünge 32a, 32b. Dabei ist der Durchmesser D' des Grundkörpers 35 der Spreizschale 2 im gespannten Zustand kleiner als der Durchmesser D des Grundkörpers 35 der Spreizschale 2 im ungespannten Zustand.

Somit gilt folgende Relation:

### Durchmesser d des Tellerelementes 15

< Durchmesser E' der gespannten Spreizschale 2 bezogen auf die radial am weitesten vorstehenden Vorsprünge 32a, 32b
< Durchmesser E der ungespannten Spreizschale 2 bezogen auf die radial am weitesten vorstehenden Vorsprünge 32a, 32b.

Noch bessere Bedingungen lassen sich durch folgende Relation erreichen:

### Durchmesser d des Tellerelementes 15

< Durchmesser D' des Grundkörpers 35 der gespannten Spreizschale 2
< Durchmesser D des Grundkörpers 35 der ungespannten Spreizschale 2

Dadurch kann die Spreizschale 2 im gespannten Zustand in den entsprechenden Knochen eingebracht werden, ohne daß die Gefahr besteht, daß das Tellerelement 15 des Setzinstruments 1 an Körperteilen hängen bleibt, da Elemente der Spreizschale 2, nämlich die radial am weitesten vorstehenden Vorsprünge 32a, 32b, über die Projektion des Tellerelementes 15, die in Einbringrichtung 13 betrachtet wird, radial hinausragen, so daß das Tellerelement 15 durch den für die Spreizschale 2 erforderlichen Kanal zum Einbringen der Spreizschale 2 einbringbar und anschließend wieder entfernbar ist.

Außerdem besteht auch beim Entfernen des Setzinstruments 1 nach dem Einbringen der Spreizschale 2 in den Knochen nicht die Gefahr, daß die Spreizschale 2 verrutscht, da durch Lösen des Tellerelements 15 entgegen der Einbringrichtung 13 relativ zu der Spreizschale 2 die Verstellkraft zum Spannen der Spreizschale 2 umfänglich gleichmäßig zurückgenommen wird, wodurch eine gleichmäßige Entspannung der einzelnen Segmente 33a bis 33d der Spreizschale 2 erfolgt. Dabei ist zu beachten, daß auf jedes der Segmente 33a bis 33d der Spreizschale 2 eine der Walzen 26a bis 26d einwirkt, da zum Beispiel für das Segment 33b der Spreizschale 2 die Walze 26b des Tellerelements 15 auf die abgeschrägte Fläche 25c der Spreizschale 2 einwirkt. Es ist allerdings auch möglich, daß, um beim Segment 33b zu bleiben, sowohl auf die abgeschrägte Fläche 25c als auch auf die abgeschrägte Fläche 25b jeweils eine Walze eines entsprechend modifizierten Tellerelements 15 einwirkt.

Fig. 4A zeigt die in Fig. 1 dargestellte Übertragungshülse 10 im Detail. Die Übertragungshülse 10 besteht aus einem rohrförmigen Grundkörper 40, der eine schlitzförmige Aussparung 41 aufweist. Durch die schlitzförmige Aussparung 41 kann der rohrförmige Grundkörper 40 auch von der Seite auf den Grundkörper 3 der Setzinstruments 1 aufgebracht werden. Der rohrförmige Grundkörper 40 der Übertragungshülse 10 weist abschnittsweise ein Innengewinde 42 auf, in das das in der Fig. 1 dargestellte Außengewinde 9 des Gewinderades 6 beim Betrieb des Setzinstruments 1 eingreift. Ferner ist an der Innenseite 43 des rohrförmigen Grundkörpers 40 der Übertragungshülse 10 ein nasenförmiger Führungskörper 44 befestigt, der im montierten Zustand des Setzinstruments 1 in die in der Fig. 2 dargestellte, sich in Längsrichtung des Grundkörpers 3 erstreckende Aussparung 30 des Grundkörpers 3 zum Führen der Übertragungshülse 10 in axialer Richtung eingreift. Somit wird ein Drehen der Übertragungshülse 10 um die Längsachse 22 verhindert.

Fig. 4B zeigt die in Fig. 4A dargestellte Übertragungshülse 10 aus der mit IV B bezeichneten Richtung. In Fig. 4B ist ein Schraubelement 45 dargestellt, das den Führungskörper 44 mit dem rohrförmigen Grundkörper 40 verbindet. Der rohrförmige Grundkörper 40 weist außerdem an seiner Stirnseite 45 eine zylinderförmige Aussparung 46 auf, in die die Distanzhülse 14 an ihrem Ende einbringbar ist, um eine vorteilhafte Abstützung und Kraftübertragung zu ermöglichen.

Fig. 5A zeigt das in der Fig. 1 dargestellte Gewinderad 6 im Detail. Das Gewinderad 6 umfaßt eine Gewindehülse 50, an der das Außengewinde 9 ausgebildet ist. Durch das Verstellrad 51 des Gewinderades 6 ist eine vorteilhafte Kraftübertragung auf das Setzinstrument 1 zum Spannen und Entspannen der Spreizschale 2 gegeben. Dabei weist das Verstellrad 51, wie es in der Fig. 5B besonders gut zu erkennen ist, mehrere an die Finger einer Hand angepaßte ergonomisch ausgebildete Aussparungen 52a bis 52f auf, durch die auch ein kräftiges Ergreifen mit einer Hand möglich ist und außerdem ein Abrutschen, selbst bei einem nassen Verstellrad 51, verhindert ist. Durch die schlitzförmige Aussparung 8' kann das Gewinderad 6 im Bereich der Flächen 7, 8 des Grundkörpers 3 montiert und demontiert werden. Im montierten Zustand des Setzinstruments 1 greift das Gewinderad 6 mit dem Außengewinde 9 in das Innengewinde 42 der Übertragungshülse 10 ein. Beim Betätigen des Gewinderads 6 im Uhrzeigersinn 12 wird ein Hub auf die Übertragungshülse 10 erzeugt, der entgegengesetzt zur Einbringrichtung 13 ist.

Das Gewinderad 6 weist außerdem das als Trapezgewinde ausgebildete Gewinde 5' auf, das im montierten Zustand des Setzinstruments 1 mit dem Gewinde 5 des Grundkörpers 3 des Setzinstruments 1 zusammenwirkt. Beim Betätigen des Gewinderads 6 im Uhrzeigersinn 12 schraubt sich das Gewinderad 6 zunehmend auf das Gewinde 5 auf, wodurch ein Hub des Gewinderades 6 in Einbringrichtung 13 erfolgt. Da die Steigung des Gewindes 5 bzw. des Gewindes 5' größer ist als die Steigung des Außengewindes 9 bzw. des Innengewindes 42, ergibt sich beim Betätigen des Gewinderades 6 im Uhrzeigersinn 12 ein resultierender Hub, der die Übertragungshülse 10 in axialer Richtung entlang der Längsachse 22 in Einbringrichtung 13 verstellt. Somit entspricht der resultierende Hub der Übertragungshülse 10 bezüglich seiner Richtung dem Hub des Gewinderades 6 relativ zu dem Grundkörper 3 des Setzinstruments 1. Dies hat den Vorteil, daß es für den operierenden Arzt sofort ersichtlich ist, in welcher Richtung die Betätigung der Übertragungshülse 10 erfolgt.

Durch den beschriebenen Mechanismus ist eine Hubübersetzungseinrichtung 54 gegeben, die eine Erhöhung der auf die Distanzhülse 14 einwirkenden Verstellkraft bedingt. Somit steht zum Spannen der Spreizschale 2 mittels des Tellerelements 15 bei einer Drehung des Gewinderads 6 im Uhrzeigersinn 12 eine große Verstellkraft zur Verfügung, die über die massiv ausgebildeten Gewinde 5, 5', 9, 42 übertragen wird.

Durch die das Gewinderad 6, die Übertragungshülse 10 und die Distanzhülse 14 umfassende Verstelleinrichtung 55 wird die von dem Arzt über das Gewinderad 6 einwirkende Betätigungskraft in eine größere Verstellkraft umgesetzt, und diese über einen gewissen Abstand mittels der Distanzhülse 14 auf das Tellerelement 15 übertragen. Somit wird das in Einbringrichtung 13 erfolgende Einbringen der Spreizschale 2 in den Knochen erheblich erleichtert. Das Verstellen des Tellerelements 15 relativ zu der an der Befestigungseinrichtung 18 befestigten Spreizschale 2 erfolgt dabei zum Spannen und Entspannen der Spreizschale 2 in axialer Richtung, d. h. entlang der Längsachse 22, so daß das seitlich nicht über die gespannte Spreizschale 2 hinausragende Tellerelement 15 beim Einbringen der Spreizschale 2 in Einbringrichtung 13 und beim anschließenden Entfernen des Setzinstruments 1 entgegen der Einbringrichtung 13 nicht an dem Knochen oder anderen Körperteilen hängen bleibt.

Die Erfindung ist nicht auf das beschriebene Ausführungsbeispiel beschränkt.

## Patentansprüche

1. Setzinstrument (1) zum Einbringen einer Spreizschale (2) in einen menschlichen oder tierischen Knochen, insbesondere einen Beckenknochen, mit
einer Befestigungseinrichtung (18) zum Befestigen der Spreizschale (2) an dem Setzinstrument (1),
einer Verstelleinrichtung (55) zum zumindest im wesentlichen axialen Verstellen eines Tellerelementes (15) des Setzinstrumentes (1) relativ zu der an der Befestigungseinrichtung (18) zu befestigenden Spreizschale (2), wobei das Tellerelement (15) beim Verstellen mit abgeschrägten Flächen (25a - 25e) der Spreizschale (2) zum Spannen der Spreizschale (2) zusammenwirkt und die in Einbringrichtung (13) betrachtete Projektion des Tellerelementes (15) nicht über die in Einbringrichtung (13) betrachtete Projektion der radial am weitesten vorstehenden Elemente (32a, 32b) der gespannten Spreizschale (2) hinausragt,
**dadurch gekennzeichnet,**
**daß** das Tellerelement (15) drehbar gelagerte Walzen (26a - 26d) umfaßt, die beim Spannen der Spreizschale (2) an den abgeschrägten Flächen (25a - 25e) der Spreizschale (2) abrollen.

2. Setzinstrument nach Anspruch 1,
**dadurch gekennzeichnet,**
**daß** die Befestigungseinrichtung (18) einen Gewindestift (16) umfaßt, auf den die Spreizschale (2) aufschraubbar ist.

3. Setzinstrument nach Anspruch 2,
**dadurch gekennzeichnet,**
**daß** der Gewindestift (16) an dem in Einbringrichtung (13) liegenden Ende (17) des Setzinstrumentes (1) angeordnet ist.

4. Setzinstrument nach einem der Ansprüche 1 bis 3,
**dadurch gekennzeichnet,**
**daß** die Verstelleinrichtung (55) ein Gewinderad (6) umfaßt, das zumindest mittelbar auf das Tellerelement (15) einwirkt.

5. Setzinstrument nach Anspruch 4,
**dadurch gekennzeichnet,**
**daß** das Gewinderad (6) mittels einer Distanzhülse (14) auf das Tellerelement (15) einwirkt.

6. Setzinstrument nach Anspruch 4 oder 5,
**dadurch gekennzeichnet,**
**daß** das Gewinderad (6) mittels einer Hubübersetzungseinrichtung (54) auf das Tellerelement (15) einwirkt,
wobei die Hubübersetzungseinrichtung (54) die auf das Tellerelement (15) einwirkende Verstellkraft vergrößert.

7. Setzinstrument nach Anspruch 6,
**dadurch gekennzeichnet,**
**daß** das Gewinderad (6) auf ein Gewinde (5) aufgebracht ist, das an dem Grundkörper (3) des Setzinstruments (1) ausgebildet ist,
**daß** das Gewinderad (6) eine Gewindehülse (50) umfaßt, an der zumindest abschnittsweise ein Außengewinde (9) ausgebildet ist,
**daß** die Gewindehülse (50) des Gewinderades (6) mit ihrem Außengewinde (9) in eine zumindest im wesentlichen axial geführte Übertragungshülse (10) eingreift,
wobei bei einer Betätigung des Gewinderades (6) der durch das Gewinde (5) vermittelte Hub der Gewindehülse (50) größer ist als der durch das Außengewinde vermittelte, in entgegengesetzter Richtung erfolgende Hub der Übertragungshülse (10).

## Claims

1. Positioning instrument (1) for inserting an extension shell (2) into a human or animal bone, in particular a pelvic bone, also a fixing device (18) for fixing the extension shell (2) on the positioning instrument (1), and a displacement device (55) for displacing a disc element (15) of the positioning instrument (1) at least essentially axially in relation to the extension shell (2), fixed on the fixing device (18),
the disc element (15) cooperating during the displacement with bevelled surfaces (25a - 25e) of the extension shell (2) in order to tighten the extension shell (2) and the projection of the disc element (15) viewed in the direction of insertion (13) not projecting beyond the projection viewed in the direction of insertion (13) of the radially furthest projecting elements (32a, 32b) of the tightened extension shell (2), **characterized in that** the disc element (15) comprises rotatably mounted rollers (26a - 26d) which, when the extension shell (2) is tightened, roll on the bevelled surfaces (25a - 25e) of the extension shell (2).

2. Positioning instrument according to claim 1, **characterized in that** the fixing device (18) comprises a grub-screw (16), on which the extension shell (2) can be screwed.

3. Positioning instrument according to claim 2, **characterized in that** the grub-screw (16) is arranged on the end of (17) of the positioning instrument (1) lying in the direction of insertion (13).

4. Positioning instrument according to any one of claims 1 to 3, **characterized in that** the displacement device (55) comprises a threaded wheel (6), which at least indirectly acts on the disc element (15).

5. Positioning instrument according to claim 4, **characterized in that** the threaded wheel (6) acts on the disc element (15) by means of a spacer (14).

6. Positioning instrument according to claim 4 or 5, **characterized in that** the threaded wheel (6) acts on the disc element (15) by means of a lifting translation device (54), whereby the lifting translation device (54) increases the displacement force acting on the disc element (15).

7. Positioning instrument according to claim 6, **characterized in that** the threaded wheel (6) is attached on a thread (5), which is formed on the base (3) of the positioning instrument (1), and **in that** the threaded wheel (6) comprises a threaded sleeve (50), on which an external thread (9) is formed at least in sections, and **in that** the threaded sleeve (50) of the threaded wheel (6) with its external thread (9) engages in an at least essentially axially arranged transmission case (10), whereby when the threaded wheel (6) is turned the lifting of the threaded sleeve (50) operated through the thread (5) is greater than the lifting of the transmission case (10) assisted by the external thread in the opposite direction.

## Revendications

1. Instrument de positionnement (1) destiné à l'insertion d'une coquille d'écartement (2) dans un os humain ou animal, en particulier dans un os du bassin, comprenant :
un moyen de fixation (18) pour fixer la coquille d'écartement (2) sur l'instrument de positionnement (1),
un dispositif de déplacement (55) pour déplacer au moins essentiellement axialement un élément en plaque (15) de l'instrument de positionnement (1) par rapport à la coquille d'écartement (2) à fixer sur le moyen de fixation (18),
dans lequel l'élément en plaque (15) coopère, lors du déplacement, avec des surfaces biseautées (25a-25e) de la coquille d'écartement (2) pour mettre sous tension la coquille d'écartement (2), et la projection de l'élément en plaque (15), observée dans la direction d'insertion (13), ne se projette pas au-delà de la projection, observée dans la direction d'insertion (13), des éléments (32a, 32b), qui dépassent radialement le plus loin, de la coquille d'écartement (2) mise sous tension,
**caractérisé en ce que**
l'élément en plaque (15) comprend des cylindres (26a-26d) montés en rotation, qui lors de la mise sous tension de la coquille d'écartement (2), roulent sur les surfaces en biseau (25a-25e) de la coquille d'écartement (2).

2. Instrument de positionnement selon la revendication 1,
**caractérisé en ce que** le moyen de fixation (18) comprend une tige filetée (16) sur laquelle la coquille d'écartement (2) peut être vissée.

3. Instrument de positionnement selon la revendication 2,
**caractérisé en ce que** la tige filetée (16) est agencée à l'extrémité (17) de l'instrument de positionnement (1) qui se trouve dans la direction d'insertion (13).

4. Instrument de positionnement selon l'une des revendications 1 à 3,
**caractérisé en ce que** le dispositif de déplacement (55) comprend une roue à pas de vis (6) qui agit au moins indirectement sur l'élément en plaque (15).

5. Instrument de positionnement selon la revendication 4,
**caractérisé en ce que** la roue à pas de vis (6) agit sur l'élément en plaque (15) au moyen d'une douille d'écartement (14).

6. Instrument de positionnement selon la revendication 4 ou 5,
**caractérisé en ce que** la roue à pas de vis (6) agit sur l'élément en plaque (15) au moyen d'un dispositif démultiplicateur de course (54),
le dispositif démultiplicateur de course (54) amplifiant la force de déplacement agissant sur l'élément en plaque (15).

7. Instrument de positionnement selon la revendication 6,
**caractérisé en ce que**
la roue à pas de vis (6) est montée sur un pas de vis (5) qui est ménagé sur le corps de base (3) de l'instrument de positionnement (1),
**en ce que** la roue à pas de vis (6) comprend une douille à pas de vis (50) sur laquelle est réalisé un pas de vis extérieur (9) au moins par tronçons,
**en ce que** la douille à pas de vis (50) de la roue à pas de vis (6) s'engage par son pas de vis extérieur (9) dans une douille de transmission (10) guidée au moins essentiellement axialement,
lors d'un actionnement de la roue à pas de vis (6), la course de la douille à pas de vis (50) provoquée par le pas de vis (5) est supérieure à la course de la douille de transmission (10) provoquée par le pas de vis extérieur et se produisant en direction opposée.
